# EUROPEAN PATENT APPLICATION

(11) **EP 4 640 231 A1**
(43) Date of publication of application: **29.10.2025**
(21) Application number: 23906814.1
(22) Date of filing: 12.12.2023
(51) Int. Cl.: A61K 38/01, A23L 33/135, A23L 33/18, A23L 33/185, A61K 35/744, A61K 35/747, A61K 36/48, A61P 43/00

(54) **ENTERAL POLYAMINE PRODUCTION AGENT**

(30) Priority: 22.12.2022 JP 2022205105
(71) Applicant: Fuji Oil Co., Ltd., Izumisano-shi, Osaka 598-8540 (JP); Kinki University, Higashi-Osaka-shi, Osaka 577-8502 (JP)
(72) Inventor: KURIHARA Shin, Kinokawa-shi, Wakayama 649-6493 (JP); HIRANO Keita, Izumisano-shi, Osaka 598-8540 (JP); SAITO Tsutomu, Izumisano-shi, Osaka 598-8540 (JP)
(74) Representative: Wimmer, Hubert
(86) International application number: PCT/JP2023/044388
(87) International publication number: WO 2024/135449

(57) **Abstract**

With an oral preparation, a polyamine is produced inside the intestine. An intestinal polyamine production agent according to the present invention contains: a bean-derived indigestible peptide formed of a decomposition residue of a bean protein material, the decomposition residue produced by decomposing the bean protein material with a protein-decomposing enzyme; and a lactic acid bacterium having an ability to decompose agmatine into putrescine.

## Description

### TECHNICAL FIELD

The present invention relates to a technology for producing a polyamine inside the intestine, and in particular, relates to a technology for producing a polyamine inside the intestine through oral intake of an indigestible peptide.

### BACKGROUND ART

In recent years, a lifespan elongation effect and a brain function enhancement effect due to intake of a polyamine have been reported (see Non-Patent Literature 1). Thus, development of a method for efficiently supplying a polyamine into the body has been desired. In view of this, various methods for producing a polyamine inside the large intestine by intestinal bacteria have been proposed (see Patent Literature 1 and Non-Patent Literature 2).

For example, an intestinal polyamine potentiator is proposed in Patent Literature 1. The intestinal polyamine potentiator contains a component that is exemplified by alanine and arginine and that promotes generation of a polyamine by intestinal bacteria. The intestinal polyamine potentiator is formed as an encapsulated agent or the like in order to deliver the component to the large intestine in an undigested state.

Regarding this proposal, the present inventors studied a method for delivering a component that promotes generation of a polyamine to the large intestine through oral intake of a so-called indigestible peptide.

The indigestible peptide is considered to reach the large intestine in an undigested state of not being decomposed into a large number of amino acids composing the indigestible peptide. Then, the indigestible peptide is considered to be decomposed into the amino acids by proteases of a wide variety of intestinal indigenous bacteria. Then, the amino acids are considered to be further utilized by the intestinal indigenous bacteria.

It is known that arginine is included among amino acids composing a soybean-derived indigestible peptide (see Non-Patent Literature 2). In addition, it was reported in Non-Patent Literature 3 that, in tests in which human feces and mice were used, arginine as a precursor of a polyamine led to increase in the amount of the polyamine to be produced by intestinal bacteria. Based on these literatures, a method for producing a polyamine was conceived. This method includes: delivering a soybean-derived indigestible peptide in an undigested state through oral intake into the large intestine so as to cause intestinal indigenous bacteria to separate arginine from the indigestible peptide in the undigested state; and furthermore, causing the intestinal indigenous bacteria to utilize the arginine.

### CITATION LIST

### [PATENT LITERATURE]

[PTL 1] Japanese Laid-Open Patent Publication No. 2015-71604

### [NON PATENT LITERATURE]

[NPL 1] R. Hirano, H. Shirasawa, and S. Kurihara, "Health-Promoting Effects of Dietary Polyamines" Med. Sci. (Basel) 9, (2021)
[NPL 2] Noriko HIGAKI, Kenji SATO, Hitoshi SUDA, Tomohiko SUZUKA, Takeo KOMORI, Tohru SAEKI, Yasushi NAKAMURA, Kozo OHTSUKI, Kimikazu IWAMI, and Ryuhei KANAMOTO, "Evidence for the Existence of a Soybean Resistant Protein That Captures Bile Acid and Stimulates Its Fecal Excretion" Biosci. Biotechnol. Biochem., 70 (12), 2844-2852, (2006)
[NPL 3] R. Kibe, S. Kurihara, Y. Sakai, H. Suzuki, T. Ooga, E. Sawaki, K. Muramatsu, A. Nakamura, A. Yamashita, Y. Kitada, M. Kakeyama, Y. Benno, and M. Matsumoto, "Upregulation of colonic luminal polyamines produced by intestinal microbiota delays senescence in mice" Sci. Rep. 4, 4548 (2014)
[NPL 4] Y. Sugiyama, M. Nara, M. Sakanaka, A. Gotoh, A. Kitakata, S. Okuda, and S. Kurihara, "Comprehensive analysis of polyamine transport and biosynthesis in the dominant human gut bacteria: potential presence of novel polyamine metabolism and transport genes" Int. J. Biochem. Cell Biol. (2017)
[NPL 5] Y. Kitada, K. Muramatsu, H. Toju, R. Kibe, Y. Benno, S. Kurihara, and M. Matsumoto, "Bioactive polyamine production by a novel hybrid system comprising multiple indigenous gut bacterial strategies" Sci. Adv. 4, eaat0062 (2018)
[NPL 6] Stefan H. M. Gorissen, Julie J. R. Crombag, Joan M. G. Senden, W. A. Huub Waterva, Jorgen Bierau, Lex B. Verdijk, and Luc J. C. van Loon, "Protein content and amino acid composition of commercially available plant-based protein isolates"
[NPL 7] Alfonso Clemente, Javier Vioque, RauA 1 SaAnchez-Vioque, Justo Pedroche, Juan Bautista, and Francisco MillaAn, "Protein quality of chickpea (Cicer arietinum L.) protein hydrolysates"
[NPL 8] <https://www.mext.go.jp/komponent/a_menu/science/detail/__icsFiles/afieldfile/2015/12/24/136 5347_2-0204-1.pdf> page of 4. Beans in Table 1 (PDF version in Japanese), Chapter 2, the section of amino acid composition tables, STANDARD TABLES OF FOOD COMPOSITION IN JAPAN - 2015 - (Seventh Revised Version) in a website of the Ministry of Education, Culture, Sports, Science and Technology, searched on December 12, 2022

### SUMMARY OF THE INVENTION

### PROBLEMS TO BE SOLVED BY THE INVENTION

However, the results of Test Example 1 described later led to a finding that, in each of human feces culture solutions corresponding to a majority of persons, the concentration of a polyamine did not increase by merely adding a soybean-derived indigestible peptide to the human feces culture solution. Meanwhile, as indicated in Test Example 2 described later, it was found that, in each of human feces culture solutions (in Test Example 1) corresponding to a majority of the persons, the amount of agmatine as a reaction intermediate generated in the course of production of putrescine from arginine increased.

Moreover, it is reported in Non-Patent Literature 4 that *Enterococcus faecalis* produces ATP by taking in agmatine inside a culture medium and discharges putrescine as a byproduct of the ATP to the outside of the cells.

Considering this, the present inventors thought that oral intake of a bacterium having an ability to decompose agmatine into putrescine in this manner together with a soybean-derived indigestible peptide might make it possible to produce putrescine inside the large intestine also in a person unable to decompose agmatine produced inside the intestine into putrescine, and hence, might make it possible to produce, inside the intestine, a polyamine exemplified by spermidine or spermine and formed from putrescine serving as a precursor.

That is, an object of the present invention is to provide a polyamine production agent containing a bean-derived indigestible peptide and a bacterium for decomposing, by being orally taken in concurrently with the indigestible peptide, agmatine derived from the indigestible peptide into putrescine.

### SOLUTION TO THE PROBLEMS

The invention made to achieve the above object is directed to an intestinal polyamine production agent containing: a bean-derived indigestible peptide formed of a decomposition residue of a bean protein material, the decomposition residue produced by decomposing the bean protein material with a protein-decomposing enzyme; and a lactic acid bacterium having an ability to decompose agmatine into putrescine.

The intestinal polyamine production agent of the present invention contains, together with the bean-derived indigestible peptide, the lactic acid bacterium for decomposing agmatine into putrescine. Consequently, the lactic acid bacterium can decompose, into putrescine, agmatine released from the indigestible peptide inside the intestine, whereby the putrescine which is a type of polyamine can be efficiently produced inside the intestine.

The bean protein material is preferably a soybean protein material. Consequently, putrescine can be more efficiently produced inside the intestine.

The lactic acid bacterium is preferably a lactic acid bacterium derived from a food product. Consequently, the polyamine production agent can be orally taken in without concern.

The lactic acid bacterium is preferably *Levilactobacillus brevis.* Consequently, putrescine can be more efficiently produced inside the intestine.

The lactic acid bacterium is preferably *Levilactobacillus brevis* derived from blue cheese. Consequently, a larger amount of putrescine can be produced inside the intestine.

### ADVANTAGEOUS EFFECTS OF THE INVENTION

As described above, the intestinal polyamine production agent of the present invention enables, through oral intake thereof, efficient production of the polyamine inside the intestine.0

### BRIEF DESCRIPTION OF THE DRAWINGS

[FIG. 1] FIG. 1 is a graph showing the results of Test Example 1 regarding research on influence that addition of a soybean-derived indigestible peptide inflicted on the concentrations of putrescine in human feces culture solutions.
[FIG. 2] FIG. 2 is a graph showing the averages of the concentrations of putrescine in the human feces culture solutions in Test Example 1 in FIG. 1.
[FIG. 3] FIG. 3 is a graph showing the results of Test Example 2 regarding research on influence that addition of the soybean-derived indigestible peptide inflicted on the concentrations of agmatine in the human feces culture solutions.
[FIG. 4] FIG. 4 is a graph showing the averages of the concentrations of agmatine in the human feces culture solutions in Test Example 2 in FIG. 3.
[FIG. 5] FIG. 5 is a graph showing the results regarding research on influence that a culture medium (Example 1), to which the soybean-derived indigestible peptide and *Enterococcus faecalis* were concurrently added, inflicted on the concentrations of putrescine in the human feces culture solutions.
[FIG. 6] FIG. 6 is a graph showing the averages of the concentrations of putrescine in the human feces culture solutions in FIG. 5.
[FIG. 7] FIG. 7 is a graph collectively showing the results of Comparative Example 2 and Example 1 regarding research on influences that addition of the indigestible peptide alone and concurrent addition of the indigestible peptide and the *E. faecalis* inflicted on the concentrations of putrescine in the human feces culture solutions, respectively.
[FIG. 8] FIG. 8 is a graph showing the results of Example 2 regarding research on influence that concurrent addition of the soybean-derived indigestible peptide and *Levilactobacillus brevis* F215 to the culture medium inflicted on the concentrations of putrescine in the human feces culture solutions.
[FIG. 9] FIG. 9 is a graph showing the averages of the concentrations of putrescine in the human feces culture solutions in Example 2 in FIG. 8.
[FIG. 10] FIG. 10 is a graph showing the results of Example 3 regarding research on influence that concurrent addition of the soybean-derived indigestible peptide and *Levilactobacillus brevis* JCM1059T to the culture medium inflicted on the concentrations of putrescine in the human feces culture solutions.
[FIG. 11] FIG. 11 is a graph showing the averages of the concentrations of putrescine in the human feces culture solutions in Example 3 in FIG. 10.
[FIG. 12] FIG. 12 is a graph collectively showing the results of Comparative Example 2 and Example 3 regarding research on influences that addition of the soybean-derived indigestible peptide alone and concurrent addition of the soybean-derived indigestible peptide and the *L. brevis* JCM1059T inflicted on the concentrations of putrescine in the human feces culture solutions, respectively.
[FIG. 13] FIG. 13 is a graph showing the results of Example 4 to Example 6 regarding research on influences that concurrent additions of pea-, chickpea-, and fava bean-derived indigestible peptides and the *Levilactobacillus brevis* F215 to the culture medium inflicted on the concentrations of putrescine in the human feces culture solutions, respectively.
[FIG. 14] FIG. 14 is a graph showing the averages of the concentrations of putrescine in the human feces culture solutions in Example 4 to Example 6 in FIG. 13.

Hereinafter, an embodiment of the present invention will be described in detail. However, the present invention is not limited to the following embodiment.

### (Intestinal Polyamine Production Agent)

An intestinal polyamine production agent according to the present invention contains: a bean-derived indigestible peptide formed from a protein-decomposing enzyme decomposition residue of a bean protein material; and a lactic acid bacterium having an ability to decompose agmatine into putrescine.

### (Beans)

Examples of the type of the bean as a raw material for the bean-derived indigestible peptide to be used in the present embodiment include plants belonging to the bean family, such as soybean, lupin bean, alfalfa, white clover, mung bean, *adzuki* bean, fava bean, pea, chickpea, common bean, lentil, and cowpea.

It is widely known that beans contain arginine (see, for example, Non-Patent Literature 6 to Non-Patent Literature 8). Among the beans, soybean, pea, chickpea, and fava bean are adopted for the following more specific description of the embodiment.

### (Soybean)

In the case of a soybean-derived indigestible peptide according to the present embodiment, the type of the soybean as a raw material for the indigestible peptide is not particularly limited as long as, after the soybean is formed into an indigestible peptide, constituent amino acids include arginine. As this soybean, a publicly-known soybean such as yellow soybean, blue soybean, black soybean, white soybean, or brown soybean may be used as appropriate.

### (Pea, Chickpea, Fava Bean)

In the case of a pea-, chickpea-, or fava bean-derived indigestible peptide according to the present embodiment, the type of the bean as a raw material for the indigestible peptide is not particularly limited as long as, after the bean is formed into an indigestible peptide, constituent amino acids include arginine. As this bean, a publicly-known pea, chickpea, or fava bean may be used as appropriate.

### (Soybean Protein Material)

A soybean protein material may be suitably used as the bean protein material.

The type of the soybean protein material is not particularly limited as long as the soybean protein material is in a state that allows treatment with a protein-decomposing enzyme to be performed in order to form an indigestible peptide. Examples of the type include unprocessed soybean, defatted soybean, soybean protein isolate, soybean protein concentrate, fractionated soybean protein, soy milk, defatted soy milk, and beans obtained by variously processing these types of soybeans. Among these types of soybeans, soybean protein isolate which is circulated and which is easily available is preferable.

### (Pea-, Chickpea-, or Fava Bean-Derived Protein Material)

A pea-, chickpea-, or fava bean-derived protein material may be used as the bean protein material. As the pea-derived protein material, the pea protein PP-CS (manufactured by Organo Food Tech Corporation) is suitably used. As the chickpea-derived protein material, ChickP S930 (manufactured by ChickP Protein Ltd.) is suitably used. As the fava bean-derived protein material, the fava bean protein ORPROTEIN (registered trademark) FP-AC (Organo Food Tech Corporation) is suitably used.

### (Protein-Decomposing Enzyme)

A protease to be used for forming the bean-derived indigestible peptide in the present embodiment may be selected as appropriate from among proteases classified into protease classes which are "metalloprotease", "acid protease", "thiol protease", and "serine protease", and may be preferably selected as appropriate from among proteases classified into protease classes which are "metalloprotease", "thiol protease", and "serine protease", regardless of whether the protease is of animal origin, plant origin, or microorganism origin. In particular, two or more types of enzymes or three or more types of enzymes belonging to different ones of these classes may be used.

This protease classification is performed through a classification method that is based on the type of an amino acid at the active center and that is ordinarily performed in the field of enzyme science. Representative examples of each of the classes are as follows. That is, representative examples of the "metalloprotease" include Bacillus neutral proteinase, Streptomyces neutral proteinase, Aspergillus neutral proteinase, THERMOASE, and the like. Representative examples of the "acid protease" include pepsin, Aspergillus acid protease, Sumizyme AP, and the like. Representative examples of the "thiol protease" include bromelain, papain, and the like. Representative examples of the "serine protease" include trypsin, chymotrypsin, subtilisin, Streptomyces alkaline protease, alcalase, Bioprase, and the like. Regarding an enzyme other than these enzymes as well, classification thereof can be ascertained according to an action pH and the reactivity with an inhibitor. The action site for a substrate significantly differs among enzymes having different active centers. Thus, the amount of "missed cleavage" is decreased, and an enzymatic decomposition product can be efficiently obtained. Also, by using enzymes of different origins (origin organisms) in combination, an enzymatic decomposition product can be further efficiently manufactured. Even in the case of enzymes belonging to a same class, when the enzymes are of different origins, the action site for a protein as a substrate also differs among the enzymes. As a result, the proportions of a dipeptide and a tripeptide can be increased. It is preferable that these proteases hardly undergo exo-activities.

### (Method for Forming Indigestible Peptide)

In the present embodiment, a reaction pH and a reaction temperature at which the bean protein material is subjected to treatment with the protease may be set according to characteristics of the protease to be used. Ordinarily, the reaction pH is set to be around an optimum pH, and the reaction temperature is set to be around an optimum temperature. The reaction temperature is about 20 to 80°C and is preferably about 40 to 60°C. After the reaction, heating is performed until arrival at a temperature (about 60 to 170°C) sufficient for deactivating the enzyme, whereby the residual enzyme is deactivated.

After the treatment with the protease, the resultant reaction liquid may be used as is or used in a concentrated state. Ordinarily, however, the reaction liquid is sterilized and subjected to spray drying, freeze-drying, or the like, to be formed into a dry powder which is then used. The sterilization is preferably performed through heating, and the temperature for the heating is preferably 110 to 170°C and further preferably 130 to 170°C. The time for the heating is preferably 3 to 20 seconds. Also, the reaction liquid may be adjusted to an arbitrarily-determined pH, and a precipitate or a suspended substance generated at the time of the pH adjustment may be removed through centrifugal separation, filtration, or the like. Furthermore, the reaction liquid may be purified with activated carbon or adsorbing resin.

### (Lactic Acid Bacterium)

The lactic acid bacterium to be used in the present embodiment is not particularly limited as long as the lactic acid bacterium has an ability to decompose agmatine into putrescine inside the large intestine. As the lactic acid bacterium, it is possible to use either: a lactic acid bacterium that is not derived from any food product and that is exemplified by an intestinal bacterium-derived lactic acid bacterium such as *Enterococcus faecalis*; or a lactic acid bacterium derived from fermented soybean paste (*miso*), yogurt, cheese, rice wine (*sake*), seasonings, and other fermented food products. Among these lactic acid bacteria, in terms of enabling safe oral intake, a lactic acid bacterium such as fermented food product-derived *Levilactobacillus brevis* is preferably used, and blue cheese-derived *Levilactobacillus brevis* is particularly preferably used.

### (Determination as to Whether Lactic Acid Bacterium Has Ability to Decompose Agmatine into Putrescine)

Determination as to whether or not a lactic acid bacterium has an ability to decompose, into putrescine, agmatine derived from a bean-derived indigestible peptide is performed as follows. That is, a lactic acid bacterium for which the determination is to be performed is added to a GAM culture medium together with human feces and a bean-derived indigestible peptide formed through the same method as in Test Example 1 described later. Then, the concentration of putrescine is measured through HPLC in the same manner as in Test Example 1. The concentrations of putrescine in 10 or more culture media formed by using feces collected from 10 or more persons are measured. Then, when the amount of increase in the average of the concentrations is determined to be statistically significant according to a p-value, the lactic acid bacterium is determined to have an ability to decompose, into putrescine, agmatine derived from the bean-derived indigestible peptide.

### (Dosage Form)

The form in which the intestinal polyamine production agent according to the present embodiment is orally taken in is not particularly limited and may be selected from among forms such as a food product, a drug, and a dietary supplement, as long as the form allows safe oral intake. When the intestinal polyamine production agent according to the present embodiment is in the form of a drug or a dietary supplement, a publicly-known dosage form such as a tablet, a powdered agent, a liquid agent, a gel tablet, or an encapsulated agent may be adopted as appropriate. Meanwhile, when the intestinal polyamine production agent according to the present embodiment is in the form of a food product, the intestinal polyamine production agent may be used as a publicly-known food product in a solid, gel, or liquid state or may be used by being mixed with such a food product.

### EXAMPLES

Next, tests performed by using Examples according to the present invention and Comparative Examples will be described in detail. However, the present invention is not limited to the following Examples.

### (Test Example 1)

By using culture solutions obtained by adding only human feces to a GAM culture medium (Comparative Example 1) and culture solutions obtained by adding the human feces and a soybean-derived indigestible peptide to the GAM culture medium (Comparative Example 2), the concentrations of putrescine produced in the culture solutions were ascertained. Here, in Comparative Example 2, external configurations in which the human feces were added to the culture medium to which the soybean-derived indigestible peptide had been added were employed in consideration of sterilization.

### (Comparative Example 1)

### <GAM Culture Medium>

11.8 g of GAM broth was added to 200 mL of Elix water, and the resultant mixture was stirred so as to completely dissolve the GAM broth. Then, the resultant solution was sterilized with an autoclave at 120°C, and then was swiftly deaerated.

### <Formation of Human Feces Samples>

Human feces collected from five males and five females, i.e., a total of 10 persons, who were aged 4 to 58 indicated in the donor list in Table 1 were dissolved in and diluted with Elix water, and the initial turbidities (OD600) of the resultant solutions were adjusted to 0.01. Consequently, human feces samples were formed.

**[Table 1]**

| No. | Age | Sex |
|---|---|---|
| 1 | 2 2 | male |
| 2 | 2 7 | male |
| 3 | 4 3 | male |
| 4 | 2 5 | female |
| 5 | 4 | female |
| 6 | 2 7 | female |
| 7 | 2 8 | female |
| 8 | 5 2 | male |
| 9 | 5 8 | male |
| 1 0 | 2 5 | female |

### <Culturing of Human Feces>

Each of the human feces samples was inoculated in the GAM culture medium, and culturing was performed at 37°C under an anaerobic condition for 24 hours. All of the experiment operations were performed in an anaerobic chamber.

### <Analysis of Polyamine in Culture Supernatant>

After the culturing by the human feces-added culture medium was completed, the concentration of the polyamine in a culture supernatant obtained through centrifugal separation was quantified through high-performance liquid chromatography (HPLC) according to Non-Patent Literature 4.

### (Comparative Example 2)

### <Soybean-Derived Indigestible Peptide>

A soybean protein isolate (FUJIPRO F) as a soybean protein material was dissolved in water having a pH of 7 such that the amount of the soybean protein isolate was 3%. Then, Bioprase (a serine protease of *Bacillus* sp. origin, available from Nagase Chemtech Co., Ltd.) was added such that the weight thereof was 1% of the weight of the protein. Then, the resultant mixture was subjected to action at a pH of 7.5 and 58°C for 60 minutes. Next, Sumizyme FP (a metalloprotease of *Aspergillus* sp. origin, available from SHINNIHON CHEMICALS Corporation) was added such that the weight thereof was 1% of the weight of the protein. Then, the resultant mixture was subjected to action at a pH of 7.5 and 58°C for 60 minutes. After the above treatments, the reaction was stopped at 90°C, and it took 20 minutes to achieve the stoppage. Then, centrifugal separation was performed so as to collect an insoluble precipitate, and the precipitate was freeze-dried, whereby a solid substance was obtained. The obtained solid substance was dissolved (suspended) in water such that the amount of the solid substance was 5%. Then, pepsin was added such that the weight thereof was 2% of the weight of the protein, the pH of the resultant mixture was adjusted to 2, and treatment was performed at 37°C for 4 hours. Then, a pancreatin was added such that the weight thereof was 1% of the weight of the protein, the pH of the resultant mixture was adjusted to 8, and treatment was performed at 37°C for 24 hours. After the above treatments, the reaction was stopped at 90°C, and it took 20 minutes to achieve the stoppage. Then, centrifugal separation was performed so as to collect an insoluble precipitate, and the precipitate was freeze-dried, whereby a soybean-derived indigestible peptide was obtained.

### <GAM + 0.5% (w/v) Soybean-Derived Indigestible Peptide Culture Medium>

11.8 g of GAM broth and 1 g (0.5% (w/v)) of the soybean-derived indigestible peptide were added to 200 mL of Elix water, and the resultant mixture was stirred so as to completely dissolve the GAM broth. The soybean-derived indigestible peptide was hardly dissolved and entered a suspended state. Then, this mixture was sterilized with an autoclave at 120°C, and then was swiftly deaerated.

### <Culturing of Human Feces>

Each of the human feces samples formed in Comparative Example 1 was inoculated in the GAM + 0.5% (w/v) soybean-derived indigestible peptide culture medium, and culturing was performed at 37°C under an anaerobic condition for 24 hours. All of the experiment operations were performed in an anaerobic chamber.

### <Analysis of Putrescine in Culture Supernatant>

After the culturing by the human feces-added culture medium was completed, the concentration of putrescine in a culture supernatant obtained through centrifugal separation was quantified through high-performance liquid chromatography (HPLC) according to Non-Patent Literature 4.

### <Results of Test Example 1>

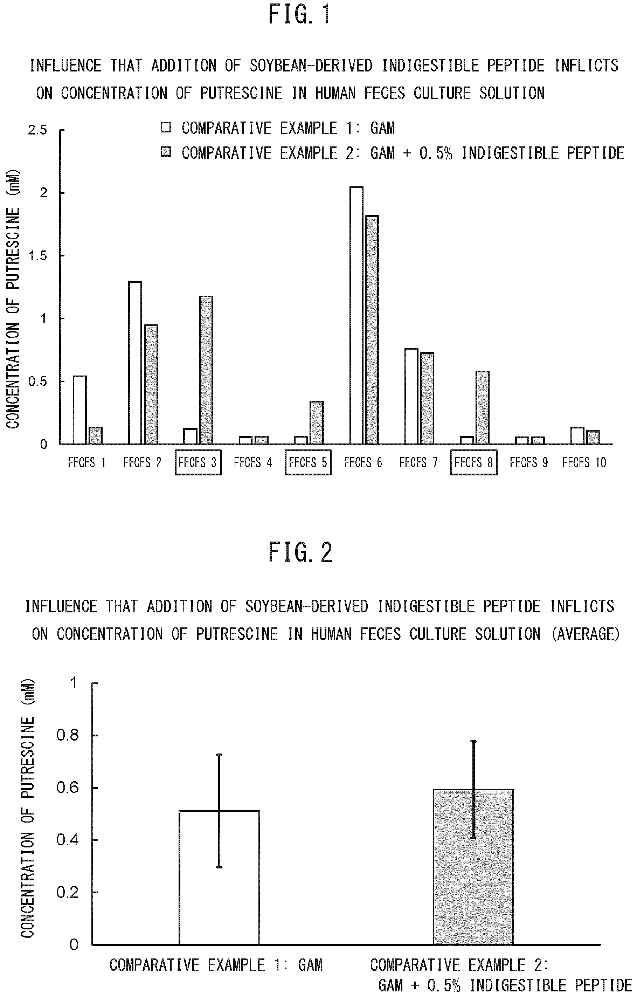

The results of Test Example 1 are shown in FIG. 1 and FIG. 2. As shown in FIG. 1, some of the feces were such that the concentrations of putrescine in Comparative Example 2 in which the indigestible peptide was added were higher than the concentrations of putrescine in Comparative Example 1 in which the indigestible peptide was not added to the human feces culture media. As shown in FIG. 1, the proportion of said feces was merely 30% of the entirety (three persons corresponding to feces 3, 5, and 8 in FIG. 1 among the 10 persons). In addition, as shown in FIG. 2, no statistically significant difference in the average of the concentrations of putrescine produced in the feces from the 10 persons was observed between Comparative Example 1 in which only the feces were added to the culture medium and Comparative Example 2 in which the indigestible peptide was added in addition to the feces to the culture medium.

### (Test Example 2)

By using the culture solutions in Comparative Example 1 (the culture solutions obtained by adding only the human feces to the GAM culture medium) and the culture solutions in Comparative Example 2 (the culture solutions obtained by adding the human feces and the soybean-derived indigestible peptide to the GAM culture medium) which were used in Test Example 1, the concentrations of agmatine in the culture solutions were measured through HPLC in the same manner as in the measurement of the concentrations of putrescine in Test Example 1.

### <Results of Test Example 2>

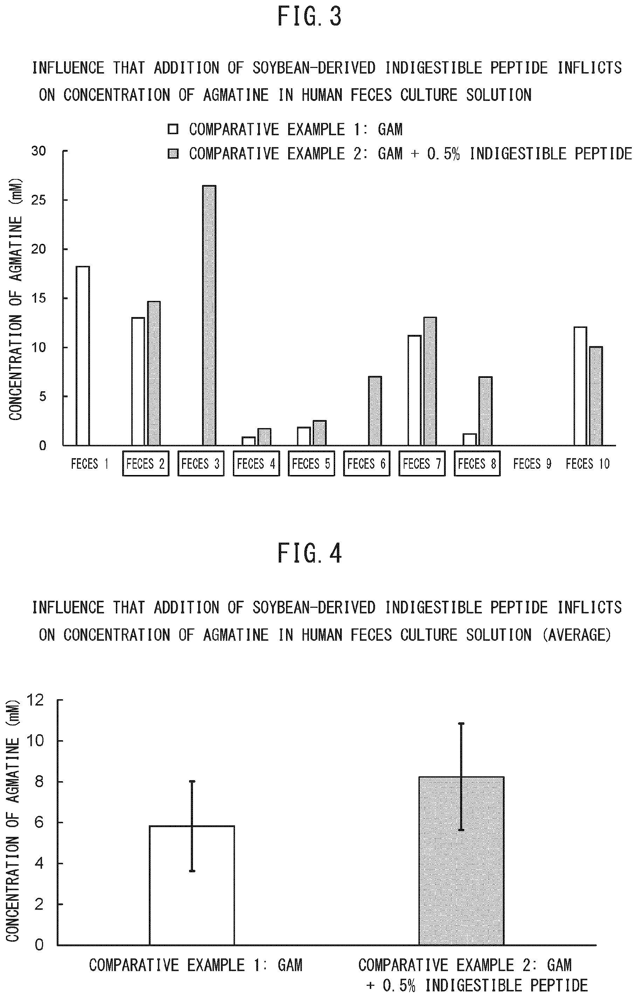

The results of Test Example 2 are shown in FIG. 3 and FIG. 4. As shown in FIG. 3, some of the feces were such that the concentrations of agmatine as a reaction intermediate generated in the course of production of putrescine from arginine in Comparative Example 2 in which the indigestible peptide was added were higher than the concentrations of the agmatine in Comparative Example 1 in which the indigestible peptide was not added to the human feces culture media. As shown in FIG. 3, the proportion of said feces was 70% of the entirety (seven persons corresponding to feces 2 to 8 in FIG. 3 among the 10 persons). In addition, as shown in FIG. 4, the average of the concentrations of the agmatine in Comparative Example 2 in which the feces and the indigestible peptide were added to the culture medium significantly increased from the average of the concentrations of the agmatine in Comparative Example 1 in which only the feces were added to the culture medium.

From the results of Test Example 1 and Test Example 2, it was considered that there were: persons in each of whom arginine released from the soybean-derived indigestible peptide having reached the inside of the large intestine was changed to putrescine; and persons in each of whom the arginine was changed only to agmatine. Therefore, a possibility of increasing putrescine inside the large intestine through oral intake of a bacterium for changing agmatine to putrescine together with the soybean-derived indigestible peptide was considered.

### (Example 1)

To each of the human feces culture media, the soybean-derived indigestible peptide and *Enterococcus faecalis* (*E. faecalis*) as a bacterium for decomposing agmatine into putrescine were added to obtain a culture medium which was then used to ascertain whether or not putrescine in the culture solution increased, in the same manner as in Test Example 1.

### <Making of Bacterium Sample>

A glycerol stock of *E. faecalis* preserved in a frozen state was picked up with a sterilized toothpick inside an anaerobic chamber and was inoculated in the GAM culture medium dispensed in a vial in advance.

The GAM culture medium and the added glycerol stock of the bacterial cells inside the vial were mixed by overturning the vial, the vial was put into a sealed container together with AnaeroPack (product number A-03 available from MITSUBISHI GAS CHEMICAL COMPANY, INC.), and the resultant mixture was cultured at 37°C for 24 hours. Before the human feces were cultured, this mixture was diluted with Elix water, and the initial turbidity (OD600) of the resultant solution was adjusted to 0.01. Consequently, a bacterium sample was made.

### <Culturing of Human Feces>

Each of the human feces samples formed in Comparative Example 1 and the bacterium sample formed from *E. faecalis* were inoculated in a GAM + 0.5% (w/v) soybean-derived indigestible peptide culture medium formed in the same manner as in Comparative Example 2, and culturing was performed at 37°C under an anaerobic condition for 24 hours. All of the experiment operations were performed in an anaerobic chamber.

### <Analysis of Putrescine in Culture Supernatant>

After the culturing by the culture medium to which the human feces, the soybean-derived indigestible peptide, and the *E. faecalis* were added was completed, the concentration of putrescine in a culture supernatant obtained through centrifugal separation was quantified through high-performance liquid chromatography (HPLC) in the same manner as in Test Example 1.

### <Results of Example 1>

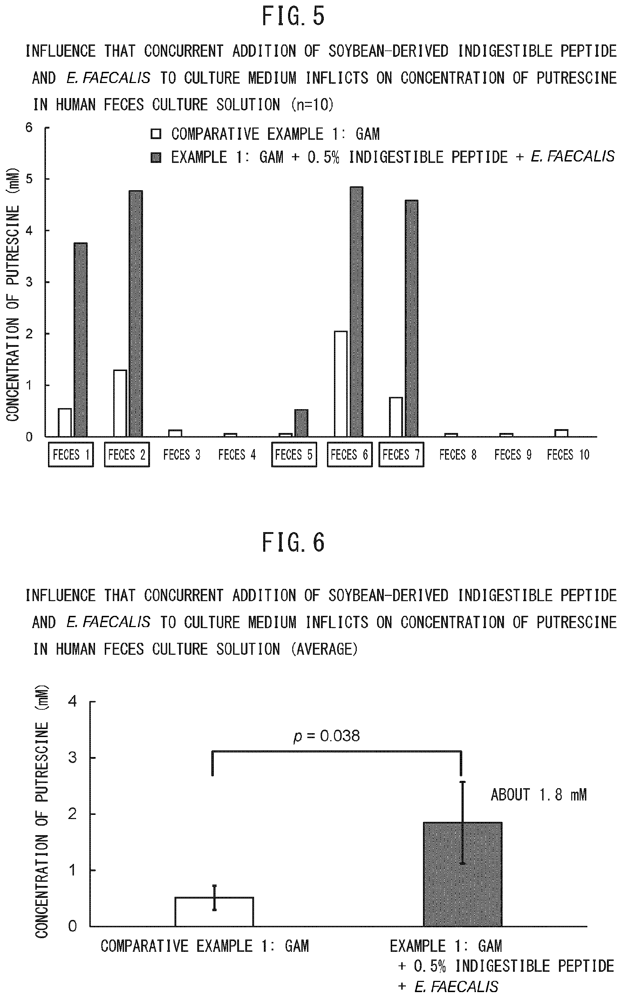

The results of Example 1 are shown in FIG. 5 to FIG. 7. As shown in FIG. 5, in 50% of the feces (five persons among the 10 persons), increase from the amounts of putrescine in the culture media according to Comparative Example 1 in which only the human feces were added to the amounts of putrescine in the culture media according to Example 1 in which the human feces, the soybean-derived indigestible peptide, and the *E. faecalis* were added was observed.

In addition, as shown in FIG. 6, the average of the concentrations of putrescine in the culture media according to Example 1 in which the human feces, the soybean-derived indigestible peptide, and the *E. faecalis* were added reached 3.6 times the average of the concentrations of putrescine in the culture media according to Comparative Example 1 in which only the human feces were added. Also, as shown in FIG. 6, the p-value was 0.038. Judging from these results, it was found that the concentrations of putrescine statistically significantly increased.

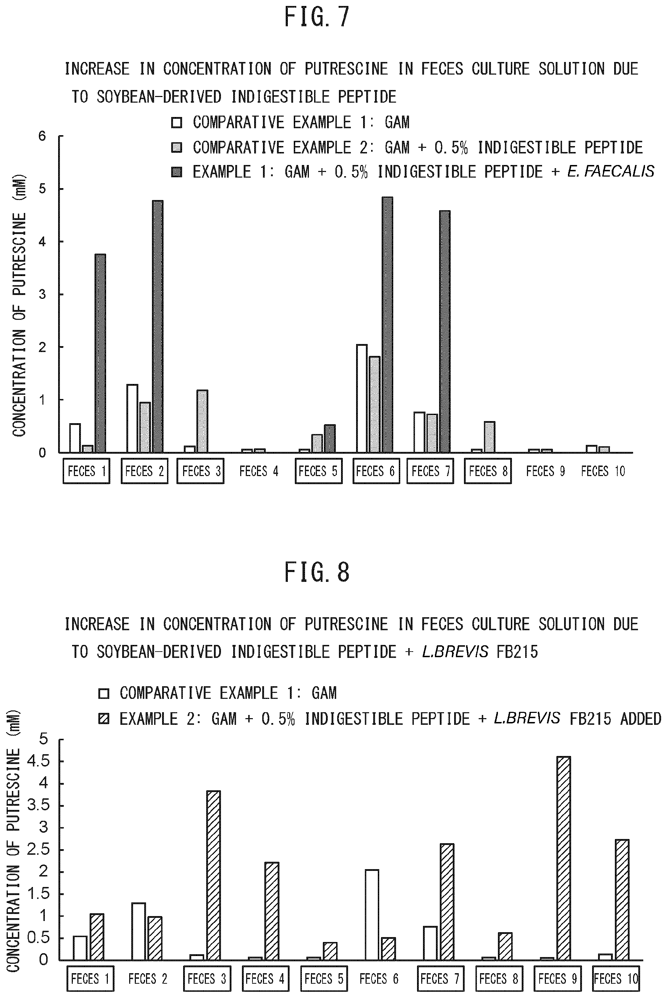

FIG. 7 collectively shows the concentrations of putrescine in Comparative Example 1 in which only the human feces were added, Comparative Example 2 in which the human feces and the indigestible peptide were added, and Example 1 in which the human feces, the indigestible peptide, and the *E. faecalis* were added. From FIG. 7, it was found that the concentration of putrescine increased in a total of 70% (seven persons among the 10 persons) of the feces through addition of the indigestible peptide alone and concurrent addition of the indigestible peptide and the *E. faecalis.*

### (Example 2)

The concentrations of putrescine in the culture solutions were measured in the same manner as in Example 1, except that each of the culture media was formed by using, instead of the *E. faecalis, Levilactobacillus brevis* collected through the following procedure as the bacterium to be added to the culture medium.

### <Collection of Bacterium>

1 to 2 g of Blue Stilton cheese was diluted with sterilized PBS so as to obtain a total volume of 10 to 20 mL, whereby a 10% (w/V) solution was obtained. At the time of weighing, kitchen scissors sterilized by spraying with 70% ethanol were used. The sample was suspended through a vortex and stirring. Next, a liquid resulting from 10²-fold, 10³-fold, 10⁴-fold, and 10⁵-fold serial dilution by using sterilized PBS was prepared. 100 µL of the diluted liquid was spread on an MRS plate and anaerobically cultured at 37°C for 120 hours. A single colony was streaked on the MRS plate and then anaerobically cultured at 37°C for 48 hours. Each of 40 strains of colonies was inoculated in 500 µL of MRS broth and anaerobically cultured at 37°C for 48 hours. Then, the resultant culture solution was subjected to centrifugal separation (at 1,900 xg for 20 minutes), and the resultant culture supernatant was subjected to a simplified quantitative method (PuO-POD-4AA-TOPS method, Anal. Biochem., 2020, 593: 113607), whereby putrescine was quantified in a simplified manner. Among the 40 strains, a bacterium indicated as having a high putrescine productivity was named FB215, and the concentration of putrescine in the culture supernatant was quantified through HPLC. Consequently, it was clarified that 150 µM of putrescine was contained.

A genome DNA extracted from the bacterial cell FB215 was used as a template, and a 16S rRNA gene was subjected to PCR amplification by using primer 1510R (5'-ACGGYTACCTTGTTACGACTT-3') and primer 7F (5'-AGAGTTTGATYMTGGCTCAG-3'). The DNA base sequence of an amplified fragment was subjected to sanger sequencing by using primer 518R (5'-GTATTACCGCGGCTGCTGG-3'), and the obtained sequence was subjected to BLAST analysis. Consequently, the sequence matched 98% or higher of the 16S rDNA of *Levilactobacillus brevis.* Therefore, hereinafter, this strain will be referred to as *Levilactobacillus brevis* FB215.

### <Results of Example 2>

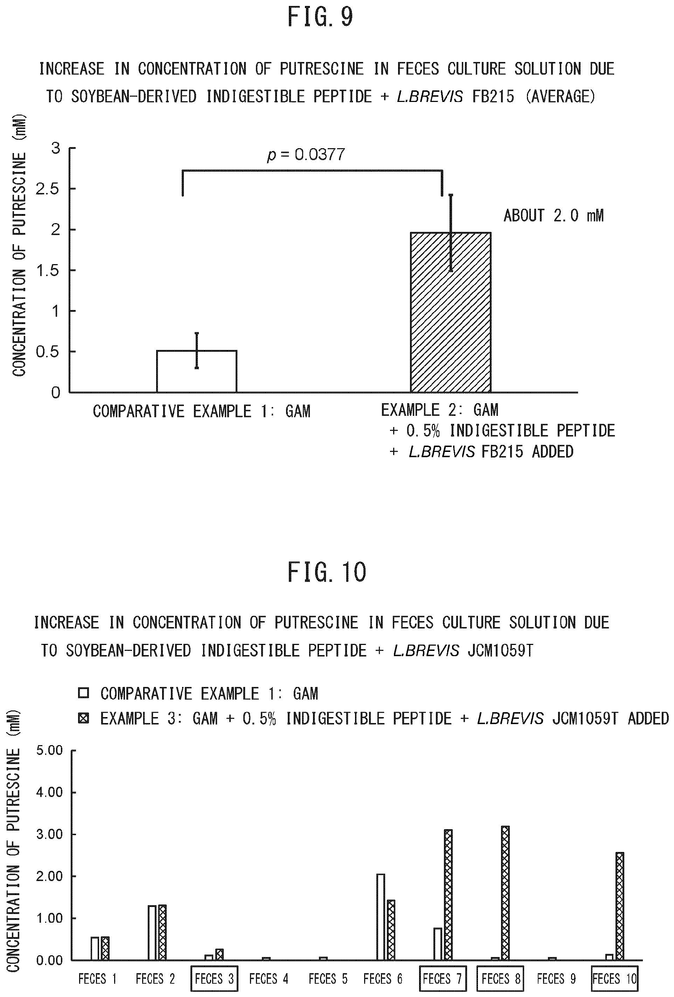

The results of Example 2 are shown in FIG. 8 and FIG. 9. As shown in FIG. 8, in 80% of the feces (eight persons among the 10 persons), the concentrations of putrescine in the culture media according to Example 2 in which the human feces, the soybean-derived indigestible peptide, and the *L. brevis* F215 were added were higher than the concentrations of putrescine in the culture media according to Comparative Example 1 in which only the human feces were added.

In addition, as shown in FIG. 9, the average of the concentrations of putrescine in the culture media according to Example 2 in which the human feces, the soybean-derived indigestible peptide, and the *L. brevis* F215 were added increased to 3.8 times the average of the concentrations of putrescine in the culture media according to Comparative Example 1 in which only the human feces were added. Also, as shown in FIG. 9, the p-value was 0.0377. Judging from these results, it was found that the concentrations of putrescine statistically significantly increased.

### (Example 3)

The concentrations of putrescine in the culture solutions were measured in the same manner as in Example 1, except that *Levilactobacillus brevis* JCM1059T was used, instead of the *E. faecalis,* as the bacterium to be added to the culture media.

### <Results of Example 3>

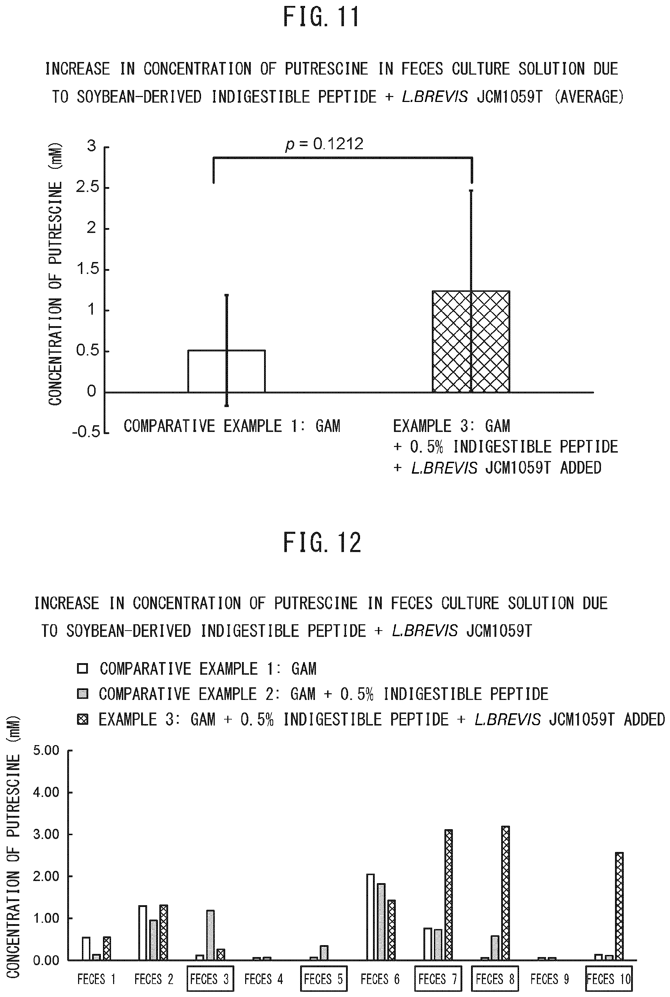

The results of Example 3 are shown in FIG. 10 and FIG. 11. As shown in FIG. 10, in 40% of the feces (four persons among the 10 persons), the concentrations of putrescine in the culture media according to Example 3 in which the human feces, the soybean-derived indigestible peptide, and the *L. brevis* JCM1059T were added were higher than the concentrations of putrescine in the culture media according to Comparative Example 1 in which only the human feces were added.

In addition, as shown in FIG. 11, it was found that, in the case of the culture media according to Example 3 in which the human feces, the soybean-derived indigestible peptide, and the *L. brevis* were added, the concentrations of putrescine in the feces from the 10 persons tended to increase as compared to the culture media according to Comparative Example 1 in which only the human feces were added, although the p-value was 0.1212.

FIG. 12 collectively shows the concentrations of putrescine in Comparative Example 1 in which only the human feces were added, Comparative Example 2 in which the human feces and the indigestible peptide were added, and Example 3 in which the human feces, the indigestible peptide, and the *L. brevis* JCM1059T were added. From FIG. 12, it was found that the concentration of putrescine increased in a total of 50% (five persons among the 10 persons) of the feces through addition of the indigestible peptide alone and concurrent addition of the indigestible peptide and the *L. brevis* JCM1059T.

### (Comparative Example 3)

The concentrations of putrescine in the culture solutions were measured through the method in Test Example 1 in the same manner as in Comparative Example 1, except that: human feces collected concurrently with the human feces used in Comparative Example 1 were caused to enter an anaerobic state and were preserved at -80°C; and then the frozen human feces were thawed and added to the GAM culture medium.

### (Example 4)

The concentrations of putrescine in the culture solutions were measured through culturing by using the *Levilactobacillus brevis* FB215 and the GAM culture medium in the same manner as in Example 2, except that: the indigestible peptide was changed to the following pea-derived indigestible peptide; and the same human feces as those in Comparative Example 3 were used.

### <Pea-Derived Indigestible Peptide>

A pea-derived indigestible peptide was formed in the same manner as in the case of the above soybean-derived indigestible peptide, except that the pea protein PP-CS (manufactured by Organo Food Tech Corporation) was used as a bean protein material.

### (Example 5)

The concentrations of putrescine in the culture solutions were measured in the same manner as in Example 4, except that the indigestible peptide was changed to the following chickpea-derived indigestible peptide.

### <Chickpea-Derived Indigestible Peptide>

A chickpea-derived indigestible peptide was formed in the same manner as in the case of the above soybean-derived indigestible peptide, except that ChickP S930 (manufactured by ChickP Protein Ltd.) was used as a bean protein material.

### (Example 6)

The concentrations of putrescine in the culture solutions were measured in the same manner as in Example 4, except that the indigestible peptide was changed to the following fava bean-derived indigestible peptide.

### <Fava Bean-Derived Indigestible Peptide>

A fava bean-derived indigestible peptide was formed in the same manner as in the case of the above soybean-derived indigestible peptide, except that the fava bean protein ORPROTEIN (registered trademark) FP-AC (Organo Food Tech Corporation) was used as a bean protein material.

### <Results of Example 4 to Example 6>

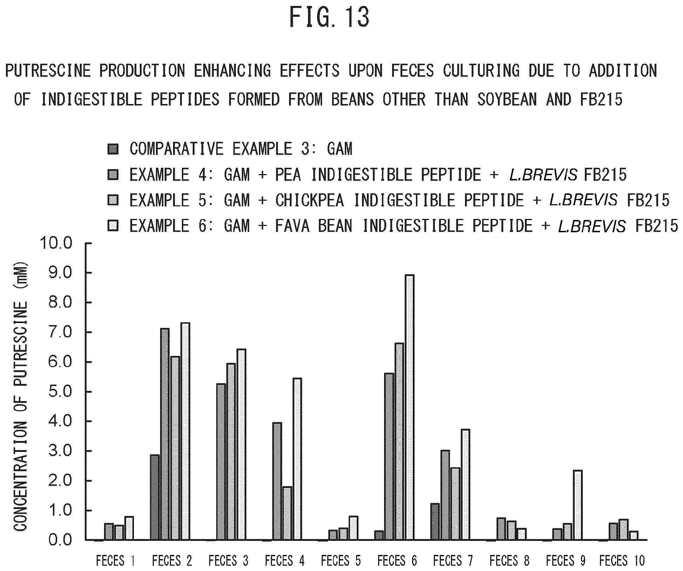

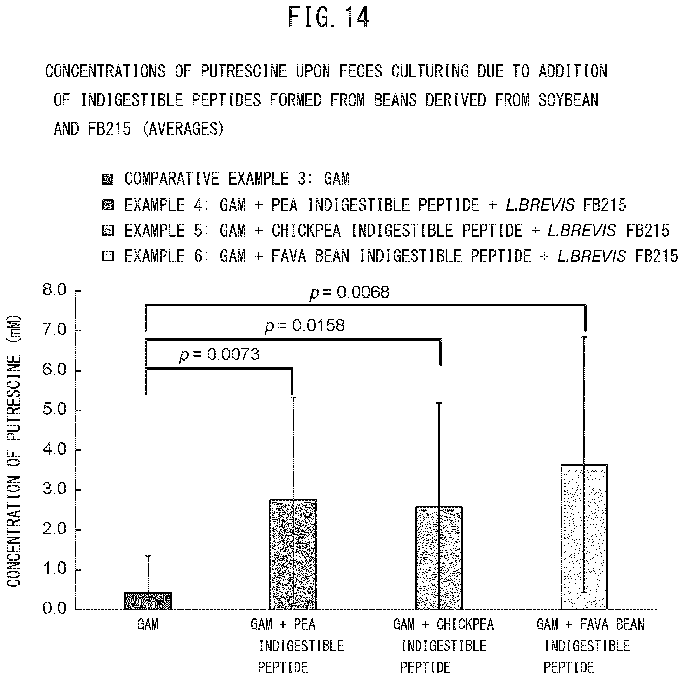

Results of Example 4 to Example 6 are shown in FIG. 13 and FIG. 14. As shown in FIG. 13, in all of the feces (10 persons among the 10 persons), the concentrations of putrescine in the culture media according to Example 4 to Example 6 in each of which the human feces, the pea-, chickpea-, or fava bean-derived indigestible peptide, and the *Levilactobacillus brevis* FB215 were added were higher than the concentrations of putrescine in the culture media according to Comparative Example 3 in which only the human feces were added.

In addition, as shown in FIG. 14, it was found that, regarding the feces from the 10 persons, the averages of the concentrations of putrescine in the culture media according to Example 4 to Example 6 in each of which the human feces, the pea-, chickpea-, or fava bean-derived indigestible peptide, and the *L. brevis* FB215 were added significantly increased respectively to 6.75 times, 6.5 times, and 9 times the culture media according to Comparative Example 3 in which only the human feces were added. In addition, it was found that the p-values in these results of Example 4 to Example 6 have small values of 0.0073, 0.0158, and 0.0068, respectively, and these results were statistically significant.

The intestinal polyamine production agent of the present invention is not limited to the above embodiment or the above Examples. For example, a lactic acid bacterium that is not derived from any food product may be used as the lactic acid bacterium for decomposing agmatine into putrescine. A lactic acid bacterium derived from a fermented food product other than cheese may be used, or a lactic acid bacterium collected from a cheese other than blue cheese may be used. As the indigestible peptide, an indigestible peptide derived from a bean other than soybean, pea, chickpea, and fava bean may be used.

## Claims

1. An intestinal polyamine production agent comprising:
a bean-derived indigestible peptide formed of a decomposition residue of a bean protein material, the decomposition residue produced by decomposing the bean protein material with a protein-decomposing enzyme; and
a lactic acid bacterium having an ability to decompose agmatine into putrescine.

2. The intestinal polyamine production agent according to claim 1, wherein the bean protein material is a soybean protein material.

3. The intestinal polyamine production agent according to claim 1, wherein the lactic acid bacterium is a lactic acid bacterium derived from a food product.

4. The intestinal polyamine production agent according to claim 3, wherein the lactic acid bacterium is *Levilactobacillus brevis.*

5. The intestinal polyamine production agent according to claim 4, wherein the lactic acid bacterium is *Levilactobacillus brevis* derived from blue cheese.

6. The intestinal polyamine production agent according to claim 1, wherein the bean protein material is a protein material derived from one of pea, chickpea, and fava bean.
